(19) Europäisches Patentamt
European Patent Office
Office européen des brevets

(11) EP 2 129 706 B1

(12) **EUROPÄISCHE PATENTSCHRIFT**

(45) Veröffentlichungstag und Bekanntmachung des Hinweises auf die Patenterteilung:
**18.05.2011 Patentblatt 2011/20**

(21) Anmeldenummer: **08717907.3**

(22) Anmeldetag: **17.03.2008**

(51) Int Cl.:
***C08J 3/24*** *(2006.01)* ***A61L 15/60*** *(2006.01)*

(86) Internationale Anmeldenummer:
**PCT/EP2008/053171**

(87) Internationale Veröffentlichungsnummer:
**WO 2008/113789 (25.09.2008 Gazette 2008/39)**

(54) **VERFAHREN ZUM BESCHICHTEN WASSERABSORBIERENDER POLYMERPARTIKEL**

METHOD FOR COATING WATER-ABSORBENT POLYMER PARTICLES

PROCÉDÉ POUR REVÊTIR DES PARTICULES POLYMÈRES ABSORBANT L'EAU

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **19.03.2007 EP 07104409**

(43) Veröffentlichungstag der Anmeldung:
**09.12.2009 Patentblatt 2009/50**

(73) Patentinhaber: **BASF SE**
**67056 Ludwigshafen (DE)**

(72) Erfinder:
- **BARTHEL, Holger**
  **68723 Oftersheim (DE)**
- **WENDKER, Martin**
  **21465 Wentorf (DE)**
- **WITT, Reiner**
  **68789 St Leon-Rot (DE)**
- **FLORE, Karin**
  **67549 Worms (DE)**

(56) Entgegenhaltungen:
**US-A1- 2004 186 244**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 129 706 B1

Printed by Jouve, 75001 PARIS (FR)

## Beschreibung

Die vorliegende Erfindung betrifft ein Verfahren zur Herstellung wasserabsorbierender Polymerpartikel, wobei in einem Mischer mittels mindestens einer thermisch isolierten und/oder begleitbeheizten Sprühdüse eine Flüssigkeit auf wasserabsorbierende Polymerpartikel aufgesprüht wird.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Zentrifugenretentionskapazität (CRC).

Zur Verbesserung der Anwendungseigenschaften, wie beispielsweise Permeabilität des gequollenen Gelbetts (SFC) in der Windel und Absorption unter Druck (AUL0.9psi), werden wasserabsorbierende Polymerpartikel im allgemeinen nachvernetzt. Dadurch steigt nur der Vernetzungsgrad der Partikeloberfläche, wodurch die Absorption unter Druck (AUL0.9psi) und die Zentrifugenretentionskapazität (CRC) zumindest teilweise entkoppelt werden können. Diese Nachvernetzung kann in wässriger Gelphase durchgeführt werden. Vorzugsweise werden aber getrocknete, gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen der wasserabsorbierenden Polymere kovalente Bindungen bilden können.

Zur weiteren Verbesserung der Permeabilität (SFC) kann die Partikeloberfläche weiter modifiziert werden, beispielsweise durch Beschichtung mit anorganischen inerten Substanzen, kationischen Polymeren und/oder Lösungen mehrwertiger Metallkationen.

EP 1 191 051 A2 beschreibt Sprühdüsen mit einem weiten Sprühwinkel zur Verwendung bei der Nachvernetzung wasserabsorbierender Polymerpartikel. Die aufzusprühende Nachvernetzerlösung weist dabei vorzugsweise eine niedrigere Temperatur auf als die vorgelegten wasserabsorbierenden Polymerpartikel.

US 2004/0181031 A1 offenbart ein Verfahren zur Nachvernetzung, wobei die wasserabsorbierenden Polymerpartikel nach der thermischen Nachvernetzung im Luftstrom abgekühlt werden.

Aufgabe der vorliegenden Erfindung war die Bereitstellung eines verbesserten Beschichtungsverfahrens für wasserabsorbierende Polymerpartikel.

Es bestand weiterhin die Aufgabe ein Beschichtungsverfahren zu finden, dass die Herstellung möglichst gleichmäßig beschichteter wasserabsorbierender Polymerpartikel mit hoher Permeabilität (SFC) und geringem Staubanteil ermöglicht.

Eine weitere Aufgabe war die Bereitstellung eines wenig störanfälligen Beschichtungsverfahren.

Gelöst wurde die Aufgabe durch ein Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) optional ein oder mehrere mit den unter a) genannten Monomeren copolymerisierbare ethylenisch und/oder allylisch ungesättigte Monomere und
d) optional ein oder mehrere wasserlösliche Polymere,

umfassend Trocknung, Mahlung und Klassierung, wobei in einem Mischer mittels mindestens einer Sprühdüse eine Flüssigkeit auf die wasserabsorbierenden Polymerpartikel aufgesprüht wird, dadurch gekennzeichnet, dass die mindestens eine Sprühdüse thermisch isoliert und/oder begleitbeheizt ist.

Thermisch isoliert bedeutet, dass die äußere Oberfläche der Sprühdüse zumindest teilweise eine weitere Materialschicht aufweist, wobei das Material der weiteren Materialschicht eine geringere Wärmeleitfähigkeit aufweist als das Material der Sprühdüse. Die Wärmeleitfähigkeit des Materials der weiteren Materialschicht bei 20°C beträgt vorzugsweise weniger als 2 $Wm^{-1}K^{-1}$, besonders bevorzugt weniger als 0,5 $Wm^{-1}K^{-1}$, ganz besonders bevorzugt weniger als 0,1 $Wm^{-1}K^{-1}$.

Begleitbeheizt bedeutet, dass der Sprühdüse zusätzlich Wärmeenergie zugeführt wird, beispielsweise mittels elektrischer Energie oder mittels eines von einem Wärmeträger durchströmten Heizmantels. Geeignete Wärmeträger sind handelsübliche Wärmeträgeröle, wie Marlotherm®, Dampf oder Warmwasser.

Eine mögliche Wärmezufuhr über einen der beim Mischen eingesetzten Einsatzstoffe, d.h. wasserabsorbierende Polymerpartikel oder zu versprühende Flüssigkeit, ist keine Begleitbeheizung im Sinne der vorliegenden Erfindung.

Geeignete Flüssigkeiten sind neben bei 23 °C flüssiger Reinstoffe auch Lösungen, Dispersionen, Emulsionen

und Schmelzen. Das erfindungsgemäße Verfahren ist insbesondere zum Beschichten mit wässrigen Lösungen oder Dispersionen geeignet.

Das erfindungsgemäße Verfahren umfasst vorzugsweise mindestens eine Nachvernetzung. In einer besonders bevorzugten Ausführungsform der vorliegenden Erfindung wird die Flüssigkeit auf nachvernetzte Polymerpartikel aufgesprüht.

Das erfindungsgemäße Verfahren ist wenig störanfällig und eignet sich daher besonders für kontinuierliche Mischer.

Der Füllgrad der Mischer beträgt vorzugsweise von 30 bis 80 %, besonders bevorzugt von 40 bis 75 %, ganz besonders bevorzugt von 50 bis 70 %.

Ein zu hoher Wassergehalt erhöht die Agglomerationsneigung der wasserabsorbierenden Polymerpartikel. Daher beträgt der Wassergehalt der im erfindungsgemäßen Verfahren einzusetzenden wasserabsorbierenden Polymerpartikel vorzugsweise weniger als 20 Gew.-%, besonders bevorzugt weniger als 10 Gew.-%, ganz besonders bevorzugt weniger als 1 Gew.-%.

Die Temperatur der wasserabsorbierenden Polymerpartikel beträgt vorzugsweise von 40 bis 80 °C, besonders bevorzugt von 45 bis 75 °C, ganz besonders bevorzugt von 50 bis 70 °C.

Die Temperatur der Sprühdüse ist vorzugsweise von 1 bis 20 °C, besonders bevorzugt von 2 bis 15 °C, ganz besonders bevorzugt von 5 bis 10 °C, höher als die Temperatur der wasserabsorbierenden Polymerpartikel.

Im Falle einer thermisch isolierten Sprühdüse ist die Temperatur der zu versprühenden Flüssigkeit vorzugsweise von 1 bis 20 °C, besonders bevorzugt von 2 bis 15 °C, ganz besonders bevorzugt von 5 bis 10 °C, höher als die Temperatur der wasserabsorbierenden Polymerpartikel. Die Temperatur der zu versprühenden Flüssigkeit entspricht ungefähr der Temperatur der Sprühdüse.

Im Falle einer begleitbeheizten und optional thermisch isolierten Sprühdüse beträgt die Temperaturdifferenz zwischen den wasserabsorbierenden Polymerpartikeln und der aufzusprühenden Flüssigkeit vorzugsweise weniger als 20 °C, bevorzugt weniger als 10 °C, besonders bevorzugt weniger als 5 °C, ganz bevorzugt weniger als 2 °C.

Die Flüssigkeit wird vorzugsweise mittels einer Zweistoffdüse, besonders bevorzugt mittels einer innenmischenden Zweistoffdüse, aufgesprüht.

Zweistoffdüsen ermöglichen eine Zerstäubung in feine Tröpfchen bzw. einen Sprühnebel. Als Zerstäubungsform wird ein kreisförmiger oder auch elliptischer Voll- oder Hohlkegel ausgebildet. Zweistoffdüsen können außenmischend oder innenmischend gestaltet werden. Bei den außenmischenden Zweistoffdüsen verlassen Flüssigkeit und Zerstäubergas den Düsenkopf über separate Öffnungen. Sie werden erst nach dem Austritt aus der Sprühdüse im Sprühstrahl gemischt. Dies ermöglicht eine im weiten Bereich unabhängige Regelung von Tropfengrößenverteilung und Durchsatz. Der Sprühkegel der Sprühdüse kann über die Luftkappenstellung eingestellt werden. Bei der innenmischenden Zweistoffdüse werden Flüssigkeit und Zerstäubergas innerhalb der Sprühdüse vermischt und das Zweiphasengemisch verlässt den Düsenkopf über dieselbe Bohrung (bzw. über mehrere parallel geschaltete Bohrungen). Bei der innenmischenden Zweistoffdüse sind die Mengen- und Druckverhältnisse stärker gekoppelt als bei der außenmischenden Sprühdüse. Geringe Veränderungen im Durchsatz führen deshalb zur Änderung der Tropfengrößenverteilung. Die Anpassung an den gewünschten Durchsatz erfolgt über den gewählten Querschnitt der Düsenbohrung.

Als Zerstäubergas kommen Pressluft, Gas oder Dampf von 0,5 bar und mehr in Frage. Die Tröpfchengröße kann individuell über das Verhältnis von Flüssigkeitsmassenstrom zu Zerstäubergasmassenstrom sowie Gas- und Flüssigkeitsdruck eingestellt werden.

Die Temperaturdifferenz zwischen der aufzusprühenden Flüssigkeit und dem Zerstäubergas beträgt vorzugsweise weniger als 20 °C, bevorzugt weniger als 10 °C, besonders bevorzugt weniger als 5 °C, ganz bevorzugt weniger als 2 °C.

Im erfindungsgemäßen Verfahren können alle dem Fachmann bekannten Mischer eingesetzt werden. Die Flüssigkeit kann sowohl in Hochgeschwindigkeitsmischern als auch in Mischern mit geringer Rührgeschwindigkeit aufgesprüht werden. Vorzugsweise werden Mischer mit bewegten Mischwerkzeugen, wie Schneckenmischer, Scheibenmischer, Pflugscharmischer, Schaufelmischer, Schraubenbandmischer,Schugi-Mischer und Durchlaufmischer, eingesetzt.

Mischer mit rotierenden Mischwerkzeugen werden gemäß der Lage der Rotationsachse in Vertikalmischer und Horizontalmischer unterteilt. Ein bevorzugter Vertikalmischer ist der Schugi-Mischer. Ein bevorzugter Horizontalmischer ist der Durchlaufmischer.

Die Verweilzeit im Vertikalmischer beträgt vorzugsweise von 0,1 bis 15 Sekunden, besonders bevorzugt von 0,25 bis 10 Sekunden, ganz besonders bevorzugt von 0,5 bis 5 Sekunden.

Die Umfangsgeschwindigkeit der Mischwerkzeuge im Vertikalmischer beträgt vorzugsweise von 2,5 bis 50 m/s, besonders bevorzugt von 5 bis 40 m/s, ganz besonders bevorzugt von 10 bis 30 m/s.

Die Verweilzeit im Horizontalmischer beträgt vorzugsweise von 1 bis 180 Minuten, besonders bevorzugt von 2 bis 60 Minuten, ganz besonders bevorzugt von 5 bis 20 Minuten.

Die Umfangsgeschwindigkeit der Mischwerkzeuge im Horizontalmischer beträgt vorzugsweise von 0,1 bis 10

m/s, besonders bevorzugt von 0,5 bis 5 m/s, ganz besonders bevorzugt von 0,75 bis 2,5 m/s.

Die wasserabsorbierenden Polymerpartikel werden im Horizontalmischer mit einer Geschwindigkeit bewegt, die einer Froude-Zahl von vorzugsweise 0,01 bis 6, besonders bevorzugt 0,05 bis 3, ganz besonders bevorzugt 0,1 bis 0,7, entspricht.

Für Mischer mit horizontal gelagertem Mischwerkzeugen ist die Froude-Zahl wie folgt definiert:

$$Fr = \frac{\omega^2 r}{g}$$

mit

r: Radius des Mischwerkzeugs
w: Kreisfrequenz
g: Erdbeschleunigung:

In einer besonders bevorzugten Ausführungsform wird die Flüssigkeit unterhalb der Produktbettoberfläche der bewegten Polymerpartikelschicht versprüht, vorzugsweise mindestens 10 mm, besonders bevorzugt mindestens 50 mm, ganz besonders bevorzugt mindestens 100 mm, d.h. die Sprühdüse taucht in das Produktbett ein.

Die Produktbettoberfläche ist die Grenzfläche, die sich zwischen den im Mischer bewegten wasserabsorbierenden Polymerpartikeln und der überlagernden Atmosphäre einstellt.

Im Horizontalmischer beträgt der Winkel zwischen der Mischerachse und der Zuführung der Sprühdüse vorzugsweise ca. 90°. Die Flüssigkeit kann senkrecht von oben zugeführt werden. Ein Zuführung schräg von der Seite ist ebenfalls möglich, wobei der Winkel gegenüber der Senkrechten vorzugsweise zwischen 60 und 90°, besonders bevorzugt zwischen 70 und 85°, ganz besonders bevorzugt zwischen 75 und 82,5°, liegt. Die schräge Anordnung der Zuführung ermöglicht die Verwendung kürzerer Zuführungen und damit geringere mechanische Belastungen während des Betriebs des Mischers.

In einer besonders bevorzugten Ausführungsform befindet sich die Sprühdüse unterhalb der Rotationsachse und sprüht in Rotationsrichtung. Durch diese Anordnung werden die beschichteten wasserabsorbierenden Polymerpartikel optimal von der Sprühdüse weggefördert. In Kombination mit der schrägen Anordnung ist es auch möglich die Sprühdüse während des Betriebs des Mischers auszutauschen, ohne dass Produkt austritt.

Geeignete Flüssigkeiten sind beispielsweise Dispersionen anorganischer inerter Substanzen, Lösungen oder Dispersionen kationischer Polymere, Lösungen zwei- oder mehrwertiger Metallkationen, sowie Polyole oder deren Lösungen.

Die Monomeren a) sind vorzugsweise wasserlöslich, d.h. die Löslichkeit in Wasser bei 23 °C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

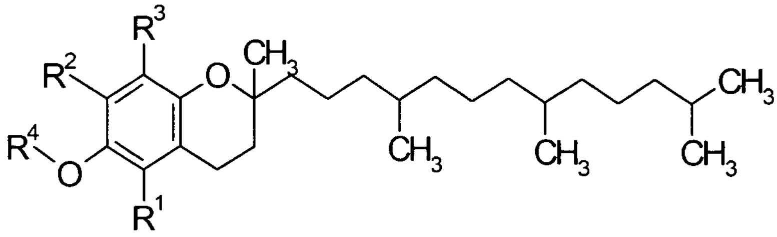

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 100 und 1000 aufweist.

Besonders vorteilhafte Vernetzer b) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins.

Die Menge an Vernetzer b) beträgt vorzugsweise 0,01 bis 15 Gew.-%, besonders bevorzugt 0,5 bis 10 Gew.-%, ganz besonders bevorzugt 1 bis 5 Gew.-%, jeweils bezogen auf die Monomerlösung.

Mit den ethylenisch ungesättigten, säuregruppentragenden Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Polymers sowie weitere geeignete hydrophile ethylenisch ungesättigte Monomere a) werden in DE 199 41 423 A1, EP 686 650 A1, WO 2001/45758 A1 und WO 2003/104300 A1 beschrieben.

Geeignete Reaktoren sind Knetreaktoren oder Bandreaktoren. Im Kneter wird das bei der Polymerisation einer wässrigen Monomerlösung entstehende Polymergel durch beispielsweise gegenläufige Rührwellen kontinuierlich zerkleinert, wie in WO 20001/38402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Fleischwolf, Extruder oder Kneter.

Vorteilhaft wird das Hydrogel nach dem Verlassen des Polymerisationsreaktors noch bei höherer Temperatur, vorzugsweise mindestens 50 °C, besonders bevorzugt mindestes 70 °C, ganz besonders bevorzugt mindestens 80 °C, sowie vorzugsweise weniger als 100 °C, gelagert, beispielsweise in isolierten Behältern. Durch die Lagerung, üblicherweise 2 bis 12 Stunden, wird der Monomerumsatz weiter erhöht.

Bei höheren Monomerumsätzen im Polymerisationsreaktor kann die Lagerung auch deutlich verkürzt bzw. auf eine Lagerung verzichtet werden.

Die Säuregruppen der erhaltenen Hydrogele sind üblicherweise teilweise neutralisiert, vorzugsweise zu 25 bis 95 mol-%, bevorzugt zu 50 bis 80 mol-%, besonders bevorzugt zu 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen.

Die Neutralisation wird vorzugsweise auf der Stufe der Monomeren durchgeführt. Dies geschieht üblicherweise durch Einmischung des Neutralisationsmittels als wässrige Lösung, als Schmelze, oder bevorzugt auch als Feststoff. Beispielsweise kann Natriumhydroxid mit einem Wasseranteil deutlich unter 50 Gew.-% als wachsartige Masse mit einem Schmelzpunkt oberhalb 23 °C vorliegen. In diesem Fall ist eine Dosierung als Stückgut oder Schmelze bei erhöhter Temperatur möglich.

Es ist aber auch möglich die Neutralisation nach der Polymerisation auf der Stufe des Hydrogels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Wird das Hydrogel zumindest teilweise nach der Polymerisation neutralisiert, so wird das Hydrogel vorzugsweise mechanisch zerkleinert, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden.

Das Hydrogel wird dann vorzugsweise mit einem Bandtrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. WSP 230.2-05 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß um so einfacher und das Produkt um so weißer, wenn der Feststoffgehalt des Gels möglichst hoch ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern.

Das getrocknete Hydrogel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA (European Disposables and Nonwovens Association) empfohlenen Testmethode Nr. WSP 220.2-05 "Partikel size distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Die Polymerpartikel können zur weiteren Verbesserung der Eigenschaften nachvernetzt werden. Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyepoxide, wie in EP 83 022 A2, EP 543 303 A1 und EP 937 736 A2 beschrieben, di- oder polyfunktionelle Alkohole, wie in DE 33 14 019 A1, DE 35 23 617 A1 und EP 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Nachvernetzer beschrieben.

Weiterhin können auch Nachvernetzer eingesetzt werden, die zusätzliche polymerisierbare ethylenisch ungesättigte Gruppen enthalten, wie in DE 37 13 601 A1 beschrieben

Die Menge an Nachvernetzer beträgt vorzugsweise 0,01 bis 1 Gew.-%, besonders bevorzugt 0,05 bis 0,5 Gew.-%, ganz besonders bevorzugt 0,1 bis 0,2 Gew.-%, jeweils bezogen auf das Polymer.

In einer bevorzugten Ausführungsform der vorliegenden Erfindung werden zusätzlich zu den Nachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat ist bevorzugt. Außer Metallsalzen können auch Polyamine als polyvalente Kationen eingesetzt werden.

Die Einsatzmenge an polyvalentem Kation beträgt beispielsweise 0,001 bis 0,5 Gew.-%, vorzugsweise 0,005 bis 0,2 Gew.-%, besonders bevorzugt 0,02 bis 0,1 Gew.-%. jeweils bezogen auf das Polymer.

Die Nachvernetzung wird üblicherweise so durchgeführt, dass eine Lösung des Nachvernetzers auf das Hydrogel oder die trockenen Polymerpartikel aufgesprüht wird. Im Anschluss an das Aufsprühen werden die mit der Nachvernetzer beschichteten Polymerpartikel thermisch getrocknet, wobei die Nachvernetzungsreaktion sowohl vor als auch während der Trocknung stattfinden kann.

Das Aufsprühen einer Lösung des Nachvernetzers wird vorzugsweise in Mischern mit bewegten Mischwerkzeugen, wie Schneckenmischer, Scheibenmischer, Pflugscharmischer und Schaufelmischer, durchgeführt. Besonders bevorzugt sind Horizontalmischer, wie Pflugscharmischer und Schaufelmischer, ganz besonders bevorzugt sind Vertikalmischer. Geeignete Mischer sind beispielsweise Lödige-Mischer, Bepex-Mischer, Nauta-Mischer, Processall-Mischer und Schugi-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250 °C, bevorzugt 120 bis 220 °C, und besonders bevorzugt 130 bis 210 °C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten.

Anschließend kann das nachvernetzte Polymer erneut klassiert werden.

Die nachvernetzten Polymerpartikel können zur weiteren Verbesserung der Eigenschaften beschichtet werden. Geeignete Beschichtungen zur Verbesserung des Akquisitionsverhaltens sowie der Permeabilität (SFC) sind beispielsweise anorganische inerte Substanzen, organische Polymere, kationische Polymere sowie zwei- oder mehrwertige Metallkationen. Geeignete Beschichtungen zur Staubbindung sind beispielsweise Polyole.

Geeignete anorganische inerte Substanzen sind Silikate, wie Montmorillonit, Kaolinit und Talk, Zeolithe, Aktivkohlen, Polykieselsäuren, Magnesiumcarbonat, Calciumcarbonat, Bariumsulfat, Aluminiumoxid, Titandioxid und Eisen-(II)-oxid. Bevorzugt gelangen Polykieselsäuren zum Einsatz, die je nach Herstellungsart zwischen Fällungskieselsäuren und pyrogenen Kieselsäuren unterschieden werden. Beide Varianten sind unter den Namen Silica FK, Sipernat®, Wessalon® (Fällungskieselsäuren) bzw. Aerosil® (pyrogene Kieselsäuren) kommerziell erhältlich. Die anorganischen inerten Substanzen können als Dispersion in einem wässrigen oder wassermischbaren Dispergiermittel oder in Substanz eingesetzt werden.

Werden die wasserabsorbierenden Polymerpartikel mit einem anorganischen inerten Material beschichtet, so beträgt die Einsatzmenge an anorganischem inerten Material bezogen auf die wasserabsorbierenden Polymerpartikel

vorzugsweise von 0,05 bis 5 Gew.-%, besonders bevorzugt von 0,1 bis 1,5 Gew.-%, ganz besonders bevorzugt von 0,3 bis 1 Gew.-%.

Geeignete organischen Materialien sind Polyalkylmethacrylate oder Thermoplaste, wie Polyvinylchlorid.

Geeignete kationische Polymere sind Polyalkylenpolyamine, kationischen Derivate von Polyacrylamiden, Polyethylenimine und polyquartäre Amine.

Polyquartäre Amine sind beispielsweise Kondensationsprodukte aus Hexamethylendiamin, Dimethylamin und Epichlorhydrin, Kondensationsprodukte aus Dimethylamin und Epichlorhydrin, Copolymere aus Hydroxyethylcellulose und Diallyldimethylammoniumchlorid, Copolymere aus Acrylamid und $\alpha$-Methacrylyloxyethyltrimethylammoniumchlorid, Kondensationsprodukte aus Hydroxyethylcellulose, Epichlorhydrin und Trimethylamin, Homopolymere von Diallyldimethylammoniumchlorid und Additionsprodukte von Epichlorhydrin an Amidoamine. Des Weiteren können polyquartäre Amine durch Umsetzung von Dimethylsulfat mit Polymeren, wie Polyethyleniminen, Copolymere aus Vinylpyrrolidon und Dimethylaminoethylmethacrylat oder Copolymere aus Ethylmethacrylat und Diethylaminoethylmethacrylat erhalten werden. Die polyquartären Amine sind in einem breiten Molekulargewichtsbereich verfügbar.

Es ist aber auch möglich die kationische Polymere auf der Partikeloberfläche zu erzeugen, entweder durch Reagenzien, die mit sich selbst ein Netzwerk bilden können, wie Additionsprodukte von Epichlorhydrin an Polyamidoamine, oder durch die Auftragung von kationischen Polymeren, die mit einem zugesetzten Vernetzer reagieren können, wie Polyamine oder Polyimine in Kombination mit Polyepoxiden, multifunktionellen Estern, multifunktionellen Säuren oder multifunktionellen (Meth)acrylaten.

Zum Einsatz gelangen können alle multifunktionellen Amine mit primären oder sekundären Aminogruppen, wie Polyethylenimin, Polyallylamin und Polylysin. Die gemäß dem erfindungsgemäßen Verfahren versprühte Flüssigkeit enthält vorzugsweise mindestens ein Polyamin, beispielsweise Polyvinylamin.

Die kationischen Polymere können als Lösung in einem wässrigen oder wassermischbaren Lösungsmittel, als Dispersion in einem wässrigen oder wassermischbaren Dispergiermittel oder in Substanz eingesetzt werden.

Werden die wasserabsorbierenden Polymerpartikel mit einem kationischen Polymer beschichtet, so beträgt die Einsatzmenge an kationischem Polymere bezogen auf die wasserabsorbierenden Polymerpartikel vorzugsweise von 0,1 bis 15 Gew.-%, besonders bevorzugt von 0,5 bis 10 Gew.-%, ganz besonders bevorzugt von 1 bis 5 Gew.-%.

Wird ein kontinuierlicher Horizontalmischer zur Beschichtung mit einem kationischen Polymer verwendet, so beträgt die Verweilzeit der wasserabsorbierenden Polymerpartikel vor dem Aufsprühen des kationischen Polymers vorzugsweise von 2 bis 50 %, besonders bevorzugt von 5 bis 30 %, ganz besonders bevorzugt von 10 bis 25 %, der Gesamtverweilzeit im Mischer.

Geeignete zwei- oder mehrwertige Metallkationen sind $Mg^{2+}$, $Ca^{2+}$, $Al^{3+}$, $Sc^{3+}$, $Ti^{4+}$, $Mn^{2+}$, $Fe^{2+/3+}$, $Co^{2+}$, $Ni^{2+}$, $Cu^{+/2+}$, $Zn^{2+}$, $Y^{3+}$, $Zr^{4+}$, $Ag^{+}$, $La^{3+}$, $Ce^{4+}$, $Hf^{4+}$, und $Au^{+/3+}$, bevorzugte Metallkationen sind $Mg^{2+}$, $Ca^{2+}$, $Al^{3+}$, $Ti^{4+}$, $Zr^{4+}$ und $La^{3+}$, besonders bevorzugte Metallkationen sind $Al^{3+}$, $Ti^{4+}$ und $Zr^{4+}$. Die Metallkationen können sowohl allein als auch im Gemisch untereinander eingesetzt werden. Von den genannten Metallkationen sind alle Metallsalze geeignet, die eine ausreichende Löslichkeit in dem zu verwendenden Lösungsmittel besitzen. Besonders geeignet sind Metallsalze mit schwach komplexierenden Anionen, wie Chlorid, Nitrat und Sulfat. Die Metallsalze werden vorzugsweise als Lösung eingesetzt. Als Lösungsmittel für die Metallsalze können Wasser, Alkohole, Dimethylformamid, Dimethylsulfoxid sowie Mischungen davon eingesetzt werden. Besonders bevorzugt sind Wasser und Wasser/Alkohol-Mischungen, wie Wasser/Methanol oder Wasser/Propylenglykol.

Die gemäß dem erfindungsgemäßen Verfahren versprühte Flüssigkeit enthält vorzugsweise mindestens ein mehrwertiges Metallkation, beispielsweise $Al^{3+}$.

Werden die wasserabsorbierenden Polymerpartikel mit einem mehrwertiges-Metallkation beschichtet, so beträgt die Einsatzmenge an mehrwertigem Metallkation bezogen auf die wasserabsorbierenden Polymerpartikel vorzugsweise von 0,05 bis 5 Gew.-%, besonders bevorzugt von 0,1 bis 1,5 Gew.-%, ganz besonders bevorzugt von 0,3 bis 1 Gew.-%.

Wird ein kontinuierlicher Horizontalmischer zur Beschichtung mit einem kationischen Polymer und einem mehrwertigen Metallkation verwendet, so beträgt die Verweilzeit der wasserabsorbierenden Polymerpartikel vor dem Aufsprühen des mehrwertigen Metallkations vorzugsweise von 1 bis 30 %, besonders bevorzugt von 2 bis 20 %, ganz besonders bevorzugt von 5 bis 15 %, der Gesamtverweilzeit im Mischer. Vorteilhaft wird das mehrwertige Metallkation vor dem kationischen Polymer dosiert.

Besonders geeignete Polyole sind Polyethylenglykole mit einem Molekulargewicht von 400 bis 20000 g/mol, Polyglyzerin, 3- bis 100-fach ethoxylierte Polyole, wie Trimethylolpropan, Glyzerin, Sorbitol und Neopentylglykol. Besonders geeignet sind 7- bis 20-fach ethoxyliertes Glyzerin oder Trimethylolpropan, wie beispielsweise Polyol TP 70® (Perstorp AB, Perstorp, SE). Letztere haben insbesondere den Vorteil, dass sie die Oberflächenspannung eines wässrigen Extrakts der wasserabsorbierenden Polymerpartikel nur unwesentlich herabsetzen. Die Polyole werden vorzugsweise als Lösung in wässrigen oder wassermischbaren Lösungsmittel eingesetzt.

Die gemäß dem erfindungsgemäßen Verfahren versprühte Flüssigkeit enthält vorzugsweise mindestens ein

Polyol, beispielsweise Polyethylenglykol.

Werden die wasserabsorbierenden Polymerpartikel mit einem Polyol beschichtet, so beträgt die Einsatzmenge an Polyol bezogen auf die wasserabsorbierenden Polymerpartikel vorzugsweise von 0,005 bis 2 Gew.-%, besonders bevorzugt von 0,01 bis 1 Gew.-%, ganz besonders bevorzugt von 0,05 bis 0,5 Gew.-%.

Wird ein kontinuierlicher Horizontalmischer zur Beschichtung mit einem kationischen Polymer und einem Polyol verwendet, so beträgt die Verweilzeit der wasserabsorbierenden Polymerpartikel vor dem Aufsprühen des Polyols vorzugsweise von 20 bis 80%, besonders bevorzugt von 30 bis 70 %, ganz besonders bevorzugt von 40 bis 60%, der Gesamtverweilzeit im Mischer. Vorteilhaft wird das Polyol nach dem kationischen Polymer dosiert.

Die obengenannten Beschichtungen können aber auch auf nicht nachvernetzte Polymerpartikel (Grundpolymer) aufgebracht werden.

Das erfindungsgemäße Verfahren eignet sich sowohl zum Aufsprühen der Nachvernetzerlösung als auch zum Aufsprühen anderer obengenannter Flüssigkeiten auf wasserabsorbierende Polymerpartikel.

Das erfindungsgemäße Verfahren ermöglicht eine gleichmäßige Beschichtung der wasserabsorbierenden Polymerpartikel und damit die Herstellung von Polymerpartikeln mit hoher Permeabilität (SFC) und niedrigem Staubanteil.

Weiterhin entstehen in dem erfindungsgemäßen Verfahren nur wenig Agglomerate. Außerdem ist das Verfahren aufgrund der verminderten Verstopfungsneigung der erfindungsgemäß einzusetzenden Sprühdüsen weniger störanfällig.

Das erfindungsgemäße Verfahren eignet sich auch zur Beschichtung mit temperaturempfindlichen Substanzen.

Die gemäß dem erfindungsgemäßen Verfahren erhältlichen wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g.

Die gemäß dem erfindungsgemäßen Verfahren erhältlichen wasserabsorbierenden Polymerpartikel weisen eine Absorption unter Druck von 6,21 kPa (AUL0.9psi) von typischerweise mindestens 10 g/g, vorzugsweise mindestens 12 g/g, bevorzugt mindestens 14 g/g, besonders bevorzugt mindestens 16 g/g, ganz besonders bevorzugt mindestens 18 g/g, und üblicherweise nicht über 30 g/g, auf.

Die gemäß dem erfindungsgemäßen Verfahren erhältlichen wasserabsorbierenden Polymerpartikel weisen eine Flüssigkeitsweiterleitung (SFC) von typischerweise mindestens $100x10^{-7}cm^3s/g$, meist mindestens $200x10^{-7}cm^3s/g$, vorzugsweise mindestens $300x10^{-7}cm^3s/g$, bevorzugt mindestens $350x10^{-7}cm^3s/g$, besonders bevorzugt mindestens $400x10^{-7}cm^3s/g$, ganz besonders bevorzugt mindestens $450x10^{-7}cm^3s/g$, und üblicherweise nicht über $700x10^{-7}cm^3s/g$, auf.

Die wasserabsorbierenden Polymerpartikel werden mit den nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

Partikelgrößenverteilung (PSD Particle Size Distribution)

Die Partikelgrößenverteilung der wasserabsorbierenden Polymerpartikel wird analog der von der EDANA (European Disposables and Nonwovens Association) empfohlenen Testmethode Nr. WSP 220.2-05 "Particle size distribution" bestimmt.

Zentrifugenretentionskapazität (CRC Centrifuge Retention Capacity)

Die Zentrifugenretentionskapazität wird analog der von der EDANA (European Disposables and Nonwovens Association) empfohlenen Testmethode Nr. WSP 241.2-05 "Centrifuge retention capacity" bestimmt, wobei die wasserabsorbierenden Polymerpartikel vor der Messung auf den Partikelgrößenbereich von größer 300 bis 600 μm abgesiebt werden.

Absorption unter Druck (AUL0.9psi Absorbency Under Load)

Die Absorption unter einem Druck von 6,21 kPa (0,9 psi) der wasserabsorbierenden Polymerpartikel wird analog der von der EDANA (European Disposables and Nonwovens Association) empfohlenen Testmethode Nr. WSP 242.2-05 "Absorption under pressure" bestimmt, wobei 0,16 g wasserabsorbierende Polymerpartikel mit einem Partikelgrößenbereich von größer 300 bis 600 μm statt 0,9 g wasserabsorbierende Polymerpartikel, als Unterboden ein Drahtnetz mit einer Maschenweite von 149 μm statt einer Maschenweite von 36 μm und ein Gewicht mit 63 g/cm$^2$ (0,9 psi) statt eines Gewichts mit 21 g/cm$^2$ (0,3 psi) verwendet werden.

Extrahierbare 16h

Der Gehalt an extrahierbaren Bestandteilen der wasserabsorbierenden Polymerpartikel wird gemäß der von der EDANA (European Disposables and Nonwovens Association) empfohlenen Testmethode Nr. WSP 270.2-05 "Extractables" bestimmt.

Flüssigkeitsweiterleitung (SFC Saline Flow Conductivity)

Die Flüssigkeitsweiterleitung einer gequollenen Gelschicht unter Druckbelastung von 21 g/cm$^2$ (0,3 psi) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC\ [cm^3s/g] = (Fg(t{=}0)xL0)/(dxAxWP),$$

wobei Fg(t=0) der Durchfluß an NaCl-Lösung in g/s ist, der anhand einer linearen Regressionsanalyse der Daten Fg(t) der Durchflußbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$ darstellt.

Staubzahl

Die Staubzahl der wasserabsorbierenden Polymerpartikel wird mit Hilfe des Staubmessgerätes Typ DustView (Palas GmbH, Karlsruhe, DE) ermittelt.

Der mechanische Teil des Messgerätes besteht aus Einfülltrichter mit Klappe, Fallrohr und Staubgehäuse mit herausnehmbarem Staubkasten.

Bei der Bestimmung der Staubzahl werden staubende Anteile von Feststoffen, welche nach definierter Beanspruchung des Materials (freier Fall und Aufprall) entstehen, quantitativ erfasst.

Die Auswertung erfolgt optoelektronisch. Der staubende Feststoffanteil führt zur Abschwächung eines Lichtstrahls, die photometrisch erfasst wird. Die Messwertregistrierung und Auswertung erfolgt in der Steuereinheit. Dabei werden die folgenden Messwerte als Zahlenwert an der Steuereinheit angezeigt:

1. Messwert nach 0,5 Sekunden (Maximalwert)
2. Messwert nach 30 Sekunden (Staubwert)
3. Staubzahl (Summe aus Maximalwert und Staubwert)

Die Staubzahlen werden wie folgt bewertet:

| | | |
|---|---|---|
| Staubzahl | 25 - 100 | staubend bis stark staubend |
| Staubzahl | 12 - 25 | gering staubend bis staubend |
| Staubzahl | 1 - 12 | gering staubend bis quasi staubfrei |
| Staubzahl | ≤ 1 | staubfrei |

Die EDANA-Testmethoden sind beispielsweise erhältlich bei der European Disposables and Nonwovens Association, Avenue Eugène Plasky 157, B-1030 Brüssel, Belgien.

Beispiele:

Die Beispiele wurden mit handelsüblichen wasserabsorbierenden Polymerpartikeln auf Natriumacrylatbasis durchgeführt.

Derartige wasserabsorbierende Polymerpartikel sind z.B. von BASF Aktiengesellschaft (Handelsname Hy-Sorb®), von Stockhausen GmbH (Handelsname Favor®) und von Nippon Shokubai Co., Ltd. (Handelsname Aqualic®) kommerziell erhältlich.

Die verwendeten wasserabsorbierenden Polymerpartikel hatten folgendes Eigenschaftsprofil:

| | | |
|---|---|---|
| CRC: | | 26,5 g/g |
| AUL0.9psi: | | 21 g/g |
| SFC: | | $120 \times 10^{-7}$ $cm^3s/g$ |
| Extractables 16h: | | 7,8 Gew.-% |
| PSD: | >850μm | 0,7 Gew.-% |
| | 600-850μm | 31,3 Gew.-% |
| | 300-600μm | 50,5 Gew.-% |
| | 90-300μm | 17,3 Gew.-% |
| | <90μm | 0,2 Gew.-% |

Beispiel 1

Die wasserabsorbierenden Polymerpartikel wurden in einem Ruberg-Durchlaufmischer Typ DLM 350-1500 (Gebrüder Ruberg GmbH & Co KG, Nieheim, DE) mittels einer Zweistoffdüse Typ RZD1-H (Gebrüder Ruberg GmbH & Co KG, Nieheim, DE) mit eine 50 gew.-%igen wässrigen Lösung von Lupamin® 9095 (BASF Aktiengesellschaft, Ludwigshafen, DE) beschichtet. Lupamin® 9095 ist ein hochmolekulares lineares Polyvinylamin.

Der Durchlaufmischer hatte ein Mischkammervolumen von 140 l. Der Füllgrad des Durchlaufmischers betrug 60 % und die Drehzahl betrug 43 $min^{-1}$. Die Froude-Zahl der bewegten wasserabsorbierenden Polymerpartikel betrug 0,36.

Die Zweistoffdüse war horizontal eingebaut. Der Abstand von der Stirnwand des Durchlaufmischers betrug 375 mm und der horizontale Abstand des Düsenmunds von der Mischerwand betrug 50 mm. Der Düsenkopf war vollständig in die wasserabsorbierenden Polymerpartikel eingetaucht. Die Sprühdüse hatte eine elektrische Begleitheizung. Die Begleitheizung wurde so geregelt, dass die Düsentemperatur 65 bis 70 °C betrug.

Der Durchsatz an wasserabsorbierenden Polymerpartikeln betrug 180 kg/h. Die Temperatur der wasserabsorbierenden Polymerpartikel betrug 60 °C.

Der Durchsatz an Beschichtungslösung betrug 7,2 kg/h. Die Temperatur der Beschichtungslösung betrug 60 °C.

Der Durchlaufmischer wurde mehrere Stunden störungsfrei betrieben.

Die beschichteten wasserabsorbierenden Polymerpartikel wurden analysiert. Die Ergebnisse sind in der Tabelle 1 zusammengefasst.

Beispiel 2 (Vergleichsbeispiel)

Es wurde verfahren wie unter Beispiel 1. Die Zweistoffdüse wurde nicht beheizt.

Der Durchlaufmischer wurde mehrere Stunden störungsfrei betrieben.

Die beschichteten wasserabsorbierenden Polymerpartikel wurden analysiert. Die Ergebnisse sind in der Tabelle 1 zusammengefasst.

Tab. 1: Beschichtung mit Lupamin® 9095

| Beispiel | CRC | AUL0.9psi | SFC | PSD >850μm |
|---|---|---|---|---|
| 1 | 27 g/g | 19 g/g | $381 \times 10^{-7}$ $cm^3s/g$ | 0,8 Gew.-% |
| 2*) | 26 g/g | 18 g/g | $257 \times 10^{-7}$ $cm^3s/g$ | 12 Gew.-% |
| *) Vergleichsbeispiel | | | | |

Beispiel 3

Es wurde verfahren wie unter Beispiel 1. Zusätzlich wurde mittels einer zweiten Zweistoffdüse mit einer 20 gew.-%igen wässrigen Lösung von Polyethylenglykol-400 (Polyethylenglykol mit einem mittleren Molgewicht von 400 g/mol) beschichtet.
Die zweite Zweistoffdüse war ebenfalls horizontal eingebaut. Der Abstand von der Stirnwand des Durchlaufmischers betrug 750 mm und der horizontale Abstand des Düsenmunds von der Mischerwand betrug 50 mm. Der Düsenkopf war vollständig in die wasserabsorbierenden Polymerpartikel eingetaucht.
Der Durchsatz an Beschichtungslösung betrug 1,35 kg/h. Die Temperatur der Beschichtungslösung betrug 20 °C.
Der Durchlaufmischer wurde mehrere Stunden störungsfrei betrieben.
Die beschichteten wasserabsorbierenden Polymerpartikel wurden analysiert. Die Ergebnisse sind in der Tabelle 2 zusammengefasst.

Beispiel 4 (Vergleichsbeispiel)

Es wurde verfahren wie unter Beispiel 3. Die Zweistoffdüsen wurden nicht beheizt.
Der Durchlaufmischer wurde mehrere Stunden störungsfrei betrieben.
Die beschichteten wasserabsorbierenden Polymerpartikel wurden analysiert. Die Ergebnisse sind in der Tabelle 2 zusammengefasst.

Tab. 2: Beschichtung mit Lupamin® 9095 und Polyethylenglykol-400

| Beispiel | CRC | AUL0.9psi | SFC | Staubzahl |
|---|---|---|---|---|
| 3 | 26 g/g | 21 g/g | 190x10$^{-7}$ cm$^3$s/g | -**) |
| 4*) | 26 g/g | 21 g/g | 170x10$^{-7}$ cm$^3$s/g | 0,7 |
| *) Vergleichsbeispiel<br>**) unterhalb der unteren Nachweisgrenze | | | | |

Beispiel 5

Es wurde verfahren wie unter Beispiel 3. Zusätzlich wurde mittels einer dritten Zweistoffdüse mit einer 23,9 gew.-%igen wässrigen Lösung von Aluminiumsulfat beschichtet.
Die dritte Zweistoffdüse war ebenfalls horizontal eingebaut. Der Abstand von der Stirnwand des Durchlaufmischers betrug 150 mm und der horizontale Abstand des Düsenmunds von der Mischerwand betrug 50 mm. Der Düsenkopf war vollständig in die wasserabsorbierenden Polymerpartikel eingetaucht.
Der Durchsatz an Beschichtungslösung betrug 7,2 kg/h. Die Temperatur der Beschichtungslösung betrug 20 °C.
Der Durchlaufmischer wurde mehrere Stunden störungsfrei betrieben.
Die beschichteten wasserabsorbierenden Polymerpartikel wurden analysiert. Die Ergebnisse sind in der Tabelle 3 zusammengefasst.

Beispiel 6 (Vergleichsbeispiel)

Es wurde verfahren wie unter Beispiel 5. Die Zweistoffdüsen wurden nicht beheizt.
Der Durchlaufmischer wurde mehrere Stunden störungsfrei betrieben.
Die beschichteten wasserabsorbierenden Polymerpartikel wurden analysiert. Die Ergebnisse sind in der Tabelle 3 zusammengefasst.

Tab. 3: Beschichtung mit Lupamin® 9095, Polyethylenglykol-400 und Aluminiumsulfat

| Beispiel | CRC | AUL0.9psi | SFC |
|---|---|---|---|
| 5 | 23 g/g | 17 g/g | 456x10$^{-7}$ cm$^3$s/g |
| 6*) | 24 g/g | 17 g/g | 413x10$^{-7}$ cm$^3$s/g |
| *) Vergleichsbeispiel | | | |

Beispiel 7

Die wasserabsorbierenden Polymerpartikel wurden in einem Pflugschar®-Mischer Typ 5 MK (Gebrüder Lödige Maschinenbau GmbH, Parderborn, DE) mittels einer Zweistoffdüse Typ 970 S4 mit einem Durchmesser von 0,8 mm (Düsen-Schlick GmbH, Untersiemau, DE) mit eine 50 gew.-%gen wässrigen Lösung von Lupamin® 9095 (BASF Aktiengesellschaft, Ludwigshafen, DE) beschichtet. Lupamin® 9095 ist ein hochmolekulares lineares Polyvinylamin.
Im Pflugschar®-Mischer wurden 1.890 g wasserabsorbierende Polymerpartikel vorgelegt. Die Temperatur der wasserabsorbierenden Polymerpartikel betrug 60 °C. Der Füllgrad des Pflugschar-Mischers betrug 60% und die Drehzahl betrug 90 $min^{-1}$.
Die Zweistoffdüse war vertikal eingebaut. Der Abstand zu den wasserabsorbierenden Polymerpartikeln betrug 100 mm. Die Sprühdüse wurde auf 65°C vorgewärmt und isoliert. Der Vordruck des Zerstäubergases betrug 3 bar. Der Durchsatz an Zerstäubergas betrug 2 kg/h.
Innerhalb von 4 Minuten wurden 76 g Beschichtungslösung aufgesprüht und 11 Minuten nachgemischt. Der Vordruck der Beschichtungslösung betrug 0,3 bar. Die Temperatur der Beschichtungslösung betrug 65 °C.
Der Pflugschar®-Mischer wurde störungsfrei betrieben.
Die beschichteten wasserabsorbierenden Polymerpartikel wurden analysiert. Die beschichteten wasserabsorbierenden Polymerpartikel hatten eine Zentrifugenretentionskapazität (CRC) von 26 g/g, eine Absorption unter Druck (AUL0.9psi) von 19 g/g, eine Flüssigkeitsweiterleitung (SFC) von $395 \times 10^{-7}$ $cm^3s/g$ und 0,9 Gew.-% Partikel größer 850μm.

Beispiel 8 (Vergleichsbeispiel)

Es wurde verfahren wie unter Beispiel 7. Die Zweistoffdüse wurde nicht isoliert.
Ablagerungen am Düsenmund führten zu einer Ablenkung und Einengung des Sprühkegels bis hin zur Verstopfung. Die wasserabsorbierenden Polymerpartikel wurden ungleichmäßig beschichtet. Es bildeten sich viele Klumpen.

**Patentansprüche**

**1.** Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) optional ein oder mehrere mit den unter a) genannten Monomeren copolymerisierbare ethylenisch und/oder allylisch ungesättigte Monomere und
d) optional ein oder mehrere wasserlösliche Polymere,

umfassend Trocknung, Mahlung und Klassierung, wobei in einem Mischer mittels mindestens einer Sprühdüse eine Flüssigkeit auf die wasserabsorbierenden Polymerpartikel aufgesprüht wird, **dadurch gekennzeichnet, dass** die mindestens eine Sprühdüse thermisch isoliert und/oder begleitbeheizt ist.

**2.** Verfahren gemäß Anspruch 1, **dadurch gekennzeichnet, dass** das Verfahren mindestens eine Nachvernetzung umfasst.

**3.** Verfahren gemäß Anspruch 2, **dadurch gekennzeichnet, dass** die Flüssigkeit auf nachvernetzte Polymerpartikel aufgesprüht wird.

**4.** Verfahren gemäß einem der Ansprüche 1 bis 3, **dadurch gekennzeichnet, dass** ein kontinuierlicher Mischer verwendet wird.

**5.** Verfahren gemäß einem der Ansprüche 1 bis 4, **dadurch gekennzeichnet, dass** der Füllgrad des Mischers von 30 bis 80 % beträgt.

**6.** Verfahren gemäß einem der Ansprüche 1 bis 5, **dadurch gekennzeichnet, dass** die dem Mischer zugeführten wasserabsorbierenden Polymerpartikel einen Wassergehalt von weniger als 20 Gew.-% aufweisen.

7. Verfahren gemäß einem der Ansprüche 1 bis 6, **dadurch gekennzeichnet, dass** die dem Mischer zugeführten wasserabsorbierenden Polymerpartikel eine Temperatur von 40 bis 80 °C aufweisen.

8. Verfahren gemäß einem der Ansprüche 1 bis 7, **dadurch gekennzeichnet, dass** die Temperatur der Sprühdüse von 1 bis 20 °C höher ist als die Temperatur der wasserabsorbierenden Polymerpartikel.

9. Verfahren gemäß einem der Ansprüche 1 bis 8, **dadurch gekennzeichnet, dass** eine thermisch isolierte Sprühdüse verwendet wird und die Temperatur der zu versprühenden Flüssigkeit von 1 bis 20 °C höher ist als die Temperatur der wasserabsorbierenden Polymerpartikel.

10. Verfahren gemäß einem der Ansprüche 1 bis 8, **dadurch gekennzeichnet, dass** eine begleitbeheizte und optional thermisch isolierte Sprühdüse verwendet wird und die Temperaturdifferenz zwischen den wasserabsorbierenden Polymerpartikeln und der aufzusprühenden Flüssigkeit weniger als 20 °C beträgt.

11. Verfahren gemäß einem der Ansprüche 1 bis 10, **dadurch gekennzeichnet, dass** die Flüssigkeit mittels einer Zweistoffdüse aufgesprüht wird.

12. Verfahren gemäß Anspruch 11, **dadurch gekennzeichnet, dass** die Temperaturdifferenz zwischen der aufzusprühenden Flüssigkeit und dem Zerstäubergas weniger als 20 °C beträgt.

13. Verfahren gemäß einem der Ansprüche 1 bis 12, **dadurch gekennzeichnet, dass** der Mischer ein Vertikalmischer ist.

14. Verfahren gemäß Anspruch 13, **dadurch gekennzeichnet, dass** die Verweilzeit der wasserabsorbierenden Polymerpartikel im Vertikalmischer von 0,1 bis 15 Sekunden beträgt.

15. Verfahren gemäß Anspruch 13 oder 14, **dadurch gekennzeichnet, dass** die Umfangsgeschwindigkeit der Mischwerkzeuge im Vertikalmischer von 2,5 bis 50 m/s beträgt.

16. Verfahren gemäß einem der Ansprüche 1 bis 12, **dadurch gekennzeichnet, dass** der Mischer ein Horizontalmischer ist.

17. Verfahren gemäß Anspruch 16, **dadurch gekennzeichnet, dass** die Verweilzeit der wasserabsorbierenden Polymerpartikel im Horizontalmischer von 1 bis 180 Minuten beträgt.

18. Verfahren gemäß Anspruch 16 oder 17, **dadurch gekennzeichnet, dass** die Umfangsgeschwindigkeit der Mischwerkzeuge im Horizontalmischer von 0,1 bis 10 m/s beträgt.

19. Verfahren gemäß einem der Ansprüche 16 bis 18, **dadurch gekennzeichnet, dass** die wasserabsorbierenden Polymerpartikel im Horizontalmischer mit einer Geschwindigkeit bewegt werden, die einer Froude-Zahl von 0,01 bis 6 entspricht.

20. Verfahren gemäß einem der Ansprüche 1 bis 19, **dadurch gekennzeichnet, dass** die Flüssigkeit eine wässrige Lösung oder Dispersion ist.

21. Verfahren gemäß einem der Ansprüche 1 bis 20, **dadurch gekennzeichnet, dass** die Flüssigkeit mindestens ein Polyamin enthält.

22. Verfahren gemäß einem der Ansprüche 1 bis 21, **dadurch gekennzeichnet, dass** die Flüssigkeit mindestens ein Polyol enthält.

23. Verfahren gemäß einem der Ansprüche 1 bis 22, **dadurch gekennzeichnet, dass** die Flüssigkeit mindestens ein mehrwertiges Metallkation enthält.

24. Verfahren gemäß einem der Ansprüche 1 bis 23, **dadurch gekennzeichnet, dass** die wasserabsorbierenden Polymerpartikel eine Zentrifugenretentionskapazität von mindestens 15 g/g aufweisen.

## Claims

1. A process for producing water-absorbing polymer particles by polymerizing a monomer solution or suspension comprising

    a) at least one ethylenically unsaturated acid-bearing monomer which may be at least partly neutralized,
    b) at least one crosslinker,
    c) optionally one or more ethylenically and/or allylically unsaturated monomers copolymerizable with the monomers specified under a) and
    d) optionally one or more water-soluble polymers,

    comprising drying, grinding and classifying, a liquid being sprayed onto the water-absorbing polymer particles by means of at least one spray nozzle in a mixer, wherein the at least one spray nozzle is thermally insulated and/or trace-heated.

2. The process according to claim 1, which comprises at least one postcrosslinking.

3. The process according to claim 2, wherein the liquid is sprayed onto postcrosslinked polymer particles.

4. The process according to any of claims 1 to 3, wherein a continuous mixer is used.

5. The process according to any of claims 1 to 4, wherein the fill level of the mixture is from 30 to 80%.

6. The process according to any of claims 1 to 5, wherein the water-absorbing polymer particles fed to the mixer have a water content of less than 20% by weight.

7. The process according to any of claims 1 to 6, wherein the water-absorbing polymer particles fed to the mixer have a temperature of from 40 to 80°C.

8. The process according to any of claims 1 to 7, wherein the temperature of the spray nozzle is from 1 to 20°C higher than the temperature of the water-absorbing polymer particles.

9. The process according to any of claims 1 to 8, wherein a thermally insulated spray nozzle is used and the temperature of the liquid to be sprayed is from 1 to 20°C higher than the temperature of the water-absorbing polymer particles.

10. The process according to any of claims 1 to 8, wherein a trace-heated and optionally thermally insulated spray nozzle is used and the temperature difference between the water-absorbing polymer particles and the liquid to be sprayed is less than 20°C.

11. The process according to any of claims 1 to 10, wherein the liquid is sprayed by means of a two-substance nozzle.

12. The process according to claim 11, wherein the temperature difference between the liquid to be sprayed and the atomizer gas is less than 20°C.

13. The process according to any of claims 1 to 12, wherein the mixer is a vertical mixer.

14. The process according to claim 13, wherein the residence time of the water-absorbing polymer particles in the vertical mixer is from 0.1 to 15 seconds.

15. The process according to claim 13 or 14, wherein the peripheral speed of the mixing tools in the vertical mixer is from 2.5 to 50 m/s.

16. The process according to any of claims 1 to 12, wherein the mixer is a horizontal mixer.

17. The process according to claim 16, wherein the residence time of the water-absorbing polymer particles in the horizontal mixer is from 1 to 180 minutes.

18. The process according to claim 16 or 17, wherein the peripheral speed of the mixing tools in the horizontal mixer is

from 0.1 to 10 m/s.

19. The process according to any of claims 16 to 18, wherein the water-absorbing polymer particles are moved in the horizontal mixer with a speed which corresponds to a Froude number of from 0.01 to 6.

20. The process according to any of claims 1 to 19, wherein the liquid is an aqueous solution or dispersion.

21. The process according to any of claims 1 to 20, wherein the liquid comprises at least one polyamine.

22. The process according to any of claims 1 to 21, wherein the liquid comprises at least one polyol.

23. The process according to any of claims 1 to 22, wherein the liquid comprises at least one polyvalent metal cation.

24. The process according to any of claims 1 to 23, wherein the water-absorbing polymer particles have a centrifuge retention capacity of at least 15 g/g.

**Revendications**

1. Procédé pour la préparation de particules de polymère absorbant l’eau, par polymérisation d’une solution ou suspension de monomères, contenant

a) au moins un monomère à insaturation éthylénique, portant des groupes acides, qui peut être au moins partiellement neutralisé,
b) au moins un agent de réticulation,
c) en option un ou plusieurs monomères à insaturation éthylénique et/ou allylique, copolymérisables avec les monomères nommés en a) et
d) en option un ou plusieurs polymères hydrosolubles,

comprenant séchage, broyage et classification, dans un mélangeur un liquide étant appliqué sur les particules de polymère absorbant l’eau, par pulvérisation au moyen d’au moins une buse de pulvérisation, **caractérisé en ce que** ladite au moins une buse de pulvérisation est isolée thermiquement et/ou chauffée par conduction.

2. Procédé selon la revendication 1, **caractérisé en ce que** le procédé comprend au moins une post-réticulation.

3. Procédé selon la revendication 2, **caractérisé en ce que** le liquide est appliqué par pulvérisation sur des particules de polymère post-réticulées.

4. Procédé selon l’une quelconque des revendications 1 à 3, **caractérisé en ce qu’**on utilise un mélangeur continu.

5. Procédé selon l’une quelconque des revendications 1 à 4, **caractérisé en ce que** le degré de remplissage du mélangeur est de 30 à 80 %.

6. Procédé selon l’une quelconque des revendications 1 à 5, **caractérisé en ce que** les particules de polymère absorbant l’eau qui sont envoyées au mélangeur présentent une teneur en eau de moins de 20 % en poids.

7. Procédé selon l’une quelconque des revendications 1 à 6, **caractérisé en ce que** les particules de polymère absorbant l’eau qui sont envoyées au mélangeur présentent une température de 40 à 80 °C.

8. Procédé selon l’une quelconque des revendications 1 à 7, **caractérisé en ce que** la température de la buse de pulvérisation est supérieure de 1 à 20 °C à la température des particules de polymère absorbant l’eau.

9. Procédé selon l’une quelconque des revendications 1 à 8, **caractérisé en ce qu’**on utilise une buse de pulvérisation isolée thermiquement et la température du liquide à pulvériser est supérieure de 1 à 20 °C à la température des particules de polymère absorbant l’eau.

10. Procédé selon l’une quelconque des revendications 1 à 8, **caractérisé en ce qu’**on utilise une buse de pulvérisation chauffée par conduction et en option isolée thermiquement et la différence de température entre les particules de

polymère absorbant l'eau et le liquide à pulvériser est inférieure à 20 °C.

**11.** Procédé selon l'une quelconque des revendications 1 à 10, **caractérisé en ce que** le liquide est appliqué par pulvérisation au moyen d'une buse binaire.

**12.** Procédé selon la revendication 11, **caractérisé en ce que** la différence de température entre le liquide à pulvériser et le gaz de pulvérisation est inférieure à 20 °C.

**13.** Procédé selon l'une quelconque des revendications 1 à 12, **caractérisé en ce que** le mélangeur est un mélangeur vertical.

**14.** Procédé selon la revendication 13, **caractérisé en ce que** le temps de séjour des particules de polymère absorbant l'eau dans le mélangeur vertical est de 0,1 à 15 secondes.

**15.** Procédé selon la revendication 13 ou 14, **caractérisé en ce que** la vitesse périphérique des outils de mélange dans le mélangeur vertical est de 2,5 à 50 m/s.

**16.** Procédé selon l'une quelconque des revendications 1 à 12, **caractérisé en ce que** le mélangeur est un mélangeur horizontal.

**17.** Procédé selon la revendication 16, **caractérisé en ce que** le temps de séjour des particules de polymère absorbant l'eau dans le mélangeur horizontal est de 1 à 180 minutes.

**18.** Procédé selon la revendication 16 ou 17, **caractérisé en ce que** la vitesse périphérique des outils de mélange dans le mélangeur horizontal est de 0,1 à 10 m/s.

**19.** Procédé selon l'une quelconque des revendications 16 à 18, **caractérisé en ce que** les particules de polymère absorbant l'eau sont mises en mouvement dans le mélangeur horizontal à une vitesse qui correspond à un nombre de Froude de 0,01 à 6.

**20.** Procédé selon l'une quelconque des revendications 1 à 19, **caractérisé en ce que** le liquide est une solution ou dispersion aqueuse.

**21.** Procédé selon l'une quelconque des revendications 1 à 20, **caractérisé en ce que** le liquide contient au moins une polyamine.

**22.** Procédé selon l'une quelconque des revendications 1 à 21, **caractérisé en ce que** le liquide contient au moins un polyol.

**23.** Procédé selon l'une quelconque des revendications 1 à 22, **caractérisé en ce que** le liquide contient au moins un cation métallique plurivalent.

**24.** Procédé selon l'une quelconque des revendications 1 à 23, **caractérisé en ce que** les particules de polymère absorbant l'eau présentent une capacité de rétention centrifuge d'au moins 15 g/g.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1191051 A2
- US 20040181031 A1
- EP 530438 A1
- EP 547847 A1
- EP 559476 A1
- EP 632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 200232962 A2
- EP 343427 A2
- DE 19941423 A1
- EP 686650 A1
- WO 200145758 A1
- WO 2000138402 A1
- DE 3825366 A1
- US 6241928 B
- EP 83022 A2
- EP 543303 A1
- EP 937736 A2
- DE 3314019 A1
- DE 3523617 A1
- EP 450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 200331482 A1
- DE 3713601 A1
- EP 0640330 A1


### In der Beschreibung aufgeführte Nicht-Patentliteratur


- Modern Superabsorbent Polymer Technology. **F.L. Buchholz ; A.T. Graham.** Monographie. Wiley-VCH, 1998, 71-103